# EUROPEAN PATENT APPLICATION

(11) **EP 4 488 257 A1**
(43) Date of publication of application: **08.01.2025**
(21) Application number: 23020322.6
(22) Date of filing: 02.07.2023
(51) Int. Cl.: C07C 67/39, C07C 69/54

(54) **OPTIMIZED PROCESS FOR MANUFACTURING METHYL METHACRYLATE**

(71) Applicant: Lygin, Alexander, 64347 Griesheim (DE)
(72) Inventor: Lygin, Alexander, 64347 Griesheim (DE)

(57) **Abstract**

Process for catalytic oxidative esterification of methacrolein with methanol and oxygen to methyl methacrylate in the presence of a heterogeneous egg-shell catalyst comprising gold metal and an oxide of at least one second element selected from Ni, Co, Fe, Zn and/or Ti supported on a support material comprising SiOs, Al₂O₃ and at least one basic element oxide, characterized in that the process is carried out in the presence of at least one compound, comprising Ni, Co, Fe, Zn and/or Ti, which is soluble in the reaction mixture under process conditions.

## Description

### Field of the invention

The present invention relates to a process for producing methyl methacrylate (MMA) by catalytic oxidative esterification of methacrolein with methanol and oxygen.

### Background of the invention

Methyl methacrylate (MMA) is one of crucially important monomers for producing transparent poly(methyl methacrylate) and a variety of co-polymers - methacrylic polymers. There are several established processes for producing MMA, most importantly that involving conversion of acetone cyanohydrine (ACH), but also the others e.g. including esterification of methacrylic acid with methanol.

The so called "LIMA" process recently developed by Evonik/Röhm and described in detail in WO2014170223A1 and some other patent applications, is believed to be one of the most efficient and economical routes to MMA currently. This process consists of three major steps (1)-(3) shown below: (1) hydroformylation of ethylene to propionaldehyde, (2) amine-catalyzed condensation of propionaldehyde with formaldehyde to obtain methacrolein (MAL) and (3) direct oxidative esterification (DOE) of MAL with methanol and oxygen to yield MMA.

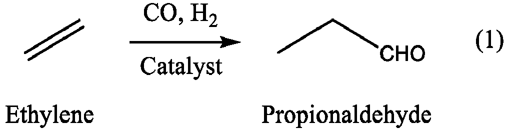

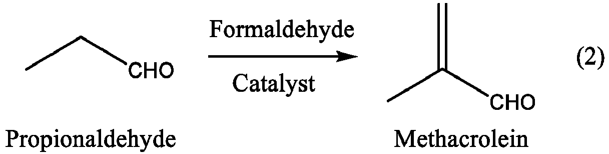

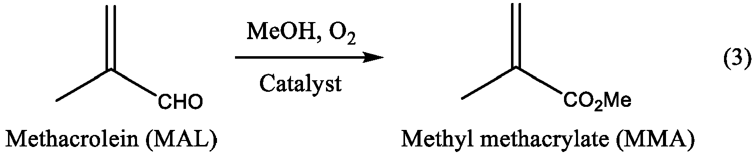

### Technical problems and solution

The catalytic oxidative esterification of aldehydes for preparation of carboxylic esters is described extensively in the prior art. Thus, U.S. Pat. No. 5,969,178 and U.S. Pat. No. 7,012,039 describe processes for continuously preparing MMA by direct oxidative esterification of MAL with methanol to give MMA with a Pd-Pb catalyst on an oxidic support.

US 20110184206A1 describes gold and nickel oxide-containing catalysts for oxidative esterification of methacrolein to methyl methacrylate with high yield and selectivity.

US9120085B2 and US9480973B2 describe a silicon-containing material consisting of Si, Al and a basic third component such as Mg, and also a metal having elevated acid resistance as a fourth component. This fourth component is Ni, Co, Zn or Fe, distributed homogeneously in the support material. Preparing a mixture of Si, Al, the basic element and the fourth component during the production of this support material ensures such a homogeneous distribution of this fourth component throughout the whole support. This support material can be used for preparing noble metal-containing catalysts. A preferred catalyst variant for the oxidative esterification of methacrolein to MMA includes an Au catalyst supported on an SiOs-Al₂O₃₋MgO-NiO material.

US2018326400A1 discloses a catalyst for direct oxidative esterification of methacrolein, comprising silica, alumina and a basic element oxide such as MgO as well as gold and at least one oxide of iron, zinc or cobalt. This catalyst shows an egg-shell distribution of gold and (Fe, Zn or Co), wherein a maximum gold concentration or a maximum (Fe, Zn or Co) concentration of the catalyst particle in an outer region which makes up a maximum of 60% of a geometric equivalent diameter is at least 1,5 times as high as the concentration thereof in a middle region of the catalyst particle. This catalyst is described to have high productivity and selectivity to MMA, as well as low mechanical abrasion of catalyst particles and low leaching of metal ions leading to long-term retention of catalyst performance.

US 20100249448A1 discloses a catalyst having an egg-shell structure with an active component comprising nickel oxide and gold nanoparticles distributed in the outer shell of the catalyst supported on a SiOs, Al₂O₃ and a basic element (e.g. Mg) oxide. This catalyst preferably has a 0.01-15 micrometer thin outer shell substantially free of active components. A thicker than 15 µm outer shell is described to lead to decrease in catalyst activity, whereas a thinner than 0.01 µm outer shell increases abrasion of the catalyst.

Thus, change of active component distribution in the catalysts for oxidative esterification of methacrolein leads to substantial changes in long-term catalyst performance and stability. Creating a specific egg-shell distribution of the active components usually boosters catalyst performance.

However, retaining catalyst form, shape and active components distribution is often problematic in a highly dynamic environment, e.g. under intense stirring conditions in a stirred tank reactor for oxidative esterification of methacrolein. Presence of reactive components such as water, acids or alkali metal hydroxides may additionally be challenging for retaining the initial catalyst characteristics.

US20190099731 A1 discloses a process for heterogeneously catalyzed reaction such as continuous oxidative esterification of methacrolein to MMA, using a reactor with two reaction zones separated by a dividing wall ensuring lower catalyst abrasion during the process. The reaction mixture flowing out of the reactor, is filtered from catalyst particles prior to its further utilization. It is suggested to use a filter with porosity 5 to 100 micrometers, preferably followed by at least one another filter with porosity of 1 to 10 micrometers for maximal retention of the catalyst in the reactor throughout the process. It is further described that the catalyst can be withdrawn from the reactor e.g. for washing or regeneration.

However, even in such a specialized reactor type, retaining catalyst form, shape and active components distribution may be challenging for the desired long process times in a continuously operated reactor.

The technical problem arising from the prior art is that of achieving continuously high yield and selectivity in the catalytic oxidative esterification of methacrolein to MMA. Particularly, it is very important to adequately deal with catalyst attrition, metal leaching and other changes in catalyst shape, structure and composition, which may be detrimental for catalytic performance in the process. More specifically, it is important to avoid active metal leaching from the outer region of catalyst particles and thus maintain an egg-shell distribution of such active components of the catalyst for a constant and high catalyst performance.

### Solution

The object of the present invention is a process for catalytic oxidative esterification of methacrolein with methanol and oxygen to methyl methacrylate in the presence of a heterogeneous egg-shell catalyst comprising gold metal and an oxide of at least one second element selected from Ni, Co, Fe, Zn and/or Ti supported on a support material comprising SiOs, Al₂O₃ and at least one basic element oxide, characterized in that the process is carried out in the presence of at least one compound, comprising Ni, Co, Fe, Zn and/or Ti, which is soluble in the reaction mixture under process conditions.

Present invention allows keeping the constantly high activity and selectivity of the employed egg-shell catalyst presumably by avoiding leaching of Ni, Co, Fe, Zn and/or Ti components from the outer shell of the catalyst. Without wishing to be bound by any theory, it is believed that leaching of insoluble or poorly soluble Ni, Co, Fe, Zn and/or Ti oxides from the outer shell of the egg-shell catalyst - in the form of soluble metal ions - may be avoided or significantly decelerated by adding the corresponding metal ions into the system and thus shifting of the corresponding solubility equilibriums of poorly soluble components of the catalyst. Hence, if Ni, Co, Fe, Zn and/or Ti compounds with certain solubility are added to the reaction mixture, less Ni, Co, Fe, Zn and/or Ti oxides are leached from the egg-shell catalyst, particularly from the outer shell of such catalysts.

This helps reserving the initial egg-shell distribution of the catalyst and thus increases high activity, selectivity, and long lifetime thereof. Another explanation for the retained egg-sell catalyst's distribution of Ni, Co, Fe, Zn and/or Ti in the inventive process may lay in the replenishment of these metals in the outer shell of the catalyst by re-deposition of these element from the soluble compound.

### The egg-shell catalyst

The term "heterogeneous catalyst" used in the present invention is conventional and clear to the person skilled in the art and refers to a catalyst present in another phase than the reaction mixture. Specifically, the "heterogeneous catalyst" referred to in the invention is a solid-phase catalyst present in the liquid phase reaction mixture.

The term "egg-shell catalyst" in the context of the present invention refers to a catalyst, wherein the concentration of the active components (gold and the second element oxide) in the outer region ("outer shell") is larger than the concentration thereof in the inner region ("core") of the catalyst.

The basic element comprised in the egg-shell catalyst may be selected from alkali metals, such as lithium (Li), sodium (Na), potassium (K); alkaline earth metals, such as magnesium (Mg), calcium (Ca), strontium (Sr), barium (Ba); rare earth metals, such as scandium (Sc), yttrium (Y), lanthanum (La), cerium (Ce), and combinations thereof. Most preferably, magnesium is used as the basic element.

The second element is preferably nickel (Ni) and/or cobalt (Co).

Most preferably, the egg-shell catalyst comprises gold, oxygen, silicon, aluminum, magnesium, cobalt and/or nickel.

The egg-shell catalyst preferably comprises, based on the total molar amount of gold, silicon, aluminum, the second element and the basic element: 0.03 mol% to 3 mol% of gold, 40 mol% to 90 mol% of silicon, 3 mol% to 40 mol% of aluminum, 0.1 mol% to 20 mol% of the second element and 2 mol% to 40 mol% of the basic element, and all these elements except for the gold and oxygen are present in the form of oxides.

The molar ratio of the second element to gold is preferably from 0.1 to 20, more preferably from 0.5 to 10, more preferably from 1 to 5.

The molar ratio of aluminum to the basic element is preferably from 0.1 to 10, more preferably from 0.3 to 5, more preferably from 0.5 to 2.

The maximum Ni, Co, Fe, Zn and/or Ti concentration in an outer region of the egg-shell catalyst particles extending from the surface of the particles to 40% of an equivalent diameter thereof is preferably at least 1,2 times, more preferably at least 1,2 times, more preferably at least 2,0 times as high as the maximum concentration of Ni, Co, Fe, Zn and/or Ti in the inner region of the egg-shell catalyst particles existing in the remaining from the outer region of the geometric equivalent diameter.

The term "equivalent diameter" used in the present invention refers to the diameter of spherical particles, or in the case of irregularly shaped particles, the diameter of a sphere having an equal volume as the particles or having a surface area equal to the surface area of the particles. Equivalent diameter is measured by measuring the mean particle diameter (volume-based) using a laser diffraction/scattering particle size distribution measuring apparatus and using the resulting value as the equivalent diameter. Alternatively, number average particle diameter as measured with a scanning electron microscope (SEM) can also be used to represent equivalent diameter.

The egg-shell catalyst preferably has a mean geometric equivalent diameter of from 10 µm to 250 µm, more preferably from 15 µm to 200 µm, more preferably from 20 µm to 150 µm, more preferably from 30 µm to 100 µm.

The thickness of the outer region in the egg-shell catalyst may be between 2 and 100 micrometers.

The egg-shell catalyst may have BET surface area of from 10 m²/g to 500 m²/g, more from 50 m²/g to 400 m²/g, more preferably from 100 m²/g to 300 m²/g. BET surface area can be determined according to DIN 9277:2014 by nitrogen adsorption in accordance with the Brunauer-Emmett-Teller method.

The egg-shell catalyst preferably has an average pore diameter of 1 nm to 50 nm. The average pore diameter can be determined by the mercury intrusion method according to DIN ISO 15901-1.

Gold is usually present in the egg-shell catalyst in the form of nanoparticles with an average diameter of below 50 nm, preferably below 20 nm, more preferably below 10 nm, more preferably from 1 nm to 10 nm. The average particle size of gold can be determined by transmission electronic microscopy (TEM). Gold can be present as individual nanoparticles and/or mixed particles comprising gold and other metals and/or metal oxides, e.g. those of Ni, Co, Fe, Zn and/or Ti.

### Spent egg-shell catalyst

The inventive process is designed to avoid or minimize the leaching of the active components of the egg-shell catalyst, particularly the oxide of the second element. However, after a long operation time due to mechanical attrition of the particles, at least a portion of the initially employed catalyst may lose its egg-shell character and become a "spent" egg-shell catalyst.

Such a spent egg-shell catalyst, wherein the maximum Ni, Co, Fe, Zn and/or Ti concentration in an outer region of the spent egg-shell catalyst particles extending from the surface of the particles to 40% of an equivalent diameter thereof is less than 1,2 times as high as the maximum concentration of Ni, Co, Fe, Zn and/or Ti in the inner region of the spent egg-shell catalyst particles existing in the remaining from the outer region of the geometric equivalent diameter, may than at least partially be separated from the egg-shell catalyst, as defined above, and withdrawn from the reaction mixture and optionally replaced with the fresh egg-shell catalyst.

The spent egg-shell catalyst is preferably separated using filtration (i.e. smaller particles of the spent catalyst go through the filter, whereas larger particles of the egg-shell catalyst are retained in the reaction mixture) and/or sedimentation (smaller particles of the spent egg-shell catalyst are withdrawn from the upper part of the sedimentation zone, whereas the larger particles of the catalyst present in the lower part of the sedimentation zone are retained in the reaction mixture).

The spent egg-shell catalyst can be regenerated and optionally returned to the oxidative esterification process. Such a regeneration can be carried out by depositing Ni, Co, Fe, Zn and/or Ti precursors and optionally gold precursors on the outer shell of the spent egg-shell catalyst, followed by thermal decomposition of such precursors so that a fresh egg-shell catalyst is formed.

### The compound

The compound may comprise Ni, Co, Fe, Zn and/or Ti, in elemental form , e.g. as nanoparticles, or preferably in oxidized, i.e. ionic form. Thus, the compound may comprise at least one salt of an inorganic or organic acid, such as nitrate, halide e.g. chloride, phosphate, sulphate, acetate, methacrylate and/or oxide, and/or hydroxide of the second element.

The compound is soluble in the reaction mixture under process conditions.

The term "soluble in the reaction mixture under process conditions" means that the second element may be detected in the filtered, homogeneous reaction mixture with typical analytical methods such as inductively coupled plasma atomic emission spectroscopy (ICP-AES), if the compound is present in the reaction mixture under process conditions.

The solubility of the compound in the reaction mixture under process conditions is preferably at least 1 ppm, more preferably at least 5 ppm, more preferably at least 10 ppm, more preferably at least 20 ppm, more preferably at least 50 ppm, more preferably at least 100 ppm, as determined by ICP-AES method.

The compound and the egg-shell catalyst preferably share at least one same second element. More preferably, all second elements present in the egg-shell catalyst, are also present in the compound.

In some embodiments of the invention, the egg-shell catalyst may itself be the compound showing certain solubility of the second element in the reaction mixture under process conditions. However preferably, the compound is not the egg-shell catalyst. Correspondingly, the compound preferably does not comprise gold metal.

The compound may be present in the reaction mixture prior to start of the process, e.g. added together with the egg-shell catalyst or preferably may be added, in portions or continuously, to the reaction mixture during the process.

If the process of oxidative esterification is carried out continuously, the compound is preferably added to the process continuously. In this case, the weight ratio of the mass of the compound added to the reactor per time unit to the sum of the masses of all reactants per time unit may be in the range between 0.1 ppm and 0.1 wt%, preferably from 1 ppm to 500 ppm.

The mass ratio of the compound to the egg-shell catalyst present in the reactor mixture during the process is preferably in the range 0.00001 to 1, more preferably 0.0001 to 0.1.

### The process

The process according to the invention may be carried out batchwise, semi-continuously or preferably continuously.

Methacrolein employed in the inventive process can preferably be obtained by a reaction of propionaldehyde with formaldehyde, i.e. the reaction according to equation (2) shown above.

This so-called Mannich reaction (2) disclosed in DE3213681A1 and elsewhere takes place in the liquid phase, in which formaldehyde and propionaldehyde are converted to methacrolein in the presence of water and in the presence of the homogeneous catalyst based at least on an acid and a base. Suitable acids are especially inorganic acids, organic monocarboxylic acids, organic dicarboxylic acids and organic polycarboxylic acids. Preference is given to using organic monocarboxylic acids such as propionic acid. Suitable bases are especially organic bases, preference being given to amines, especially secondary amines such as dimethylamine, diethylamine, methylethylamine, methylpropylamine, dipropylamine, dibutylamine, diisopropylamine, diisobutylamine, methylisopropylamine, methylisobutylamine, methyl-sec-butylamine, methyl(2-methylpentyl)amine, methyl(2-ethylhexyl)amine, pyrrolidine, piperidine, morpholine, N-methylpiperazine, N-hydroxyethylpiperazine, piperazine, hexamethyleneimine, diethanolamine, methylethanolamine, methylcyclohexylamine, methylcyclopentylamine or dicyclohexylamine. It is also possible to use mixtures of two or more bases.

Propionaldehyde employed for the synthesis of methacrolein used in the inventive process can preferably be obtained by hydroformylation of ethylene, i.e. reaction according to equation (1) shown above.

The hydroformylation in general is well known, and reference is made in this connection to standard literature, for example Kirk-Othmer Encyclopedia of Chemical Technology, John Wiley & Sons, Inc., OXO Process and Franke et al., Applied Hydroformylation, dx.doi.org/10.1021 /cr3001803, Chem. Rev. 2012, 112, 5675-5732. Catalysts are usually used for this reaction. Among the preferred catalysts are in particular compounds in which rhodium, iridium, palladium and/or cobalt is present, particular preference being given here to rhodium. Metal complexes which comprise at least one phosphorus-containing compound as ligand are typically used as catalysts. The hydroformylation of ethene uses carbon monoxide and hydrogen, usually in the form of a mixture known as synthesis gas. The composition of the synthesis gas used for the hydroformylation process can vary widely. The molar ratio of carbon monoxide and hydrogen is generally from 2:1 to 1:2, in particular about 45:55 to 50:50.

The temperature in the hydroformylation reaction is generally in the range of about 50 to 200° C., preferably about 60 to 190° C., in particular about 90 to 190° C. The reaction is preferably carried out at a pressure in the range of about 5 to 700 bar, preferably 10 to 200 bar, in particular 15 to 60 bar. The reaction pressure can be varied, depending on the activity of the hydroformylation catalyst used.

Most preferably, the sequence of processes according to equations (1), (2) and (3), i.e. hydroformylation of ethylene followed by the reaction of the obtained propionaldehyde with formaldehyde to methacrolein and oxidative esterification of MAL to MMA, is used in the present invention to obtain methyl methacrylate.

The inventive process is carried out in the presence of the compound soluble in the reaction mixture under process conditions. Therefore, reaction mixture extracted from the oxidative esterification process may contain significant amounts of Ni, Co, Fe, Zn and/or Ti fine particles (e.g. nanoparticles) and/or ions. Such Ni, Co, Fe, Zn and/or Ti particles and/or ions finely dispersed and/or dissolved in the reaction mixture exiting the reactor for carrying out oxidative esterification reaction can be trapped immediately after the reactor or further in the product workup, using a suitable trapping means, such as an ion exchange resin or an appropriate adsorbent, e.g. active carbon.

## Claims

1. Process for catalytic oxidative esterification of methacrolein with methanol and oxygen to methyl methacrylate in the presence of a heterogeneous egg-shell catalyst comprising gold metal and an oxide of at least one second element selected from Ni, Co, Fe, Zn and/or Ti supported on a support material comprising SiOs, Al₂O₃ and at least one basic element oxide, **characterized in that**
the process is carried out in the presence of at least one compound, comprising Ni, Co, Fe, Zn and/or Ti, which is soluble in the reaction mixture under process conditions.

2. The process according to claim 1, wherein the maximum Ni, Co, Fe, Zn and/or Ti concentration in an outer region of the egg-shell catalyst particles extending from the surface of the particles to 40% of an equivalent diameter thereof is at least 1,2 times as high as the maximum concentration of Ni, Co, Fe, Zn and/or Ti in the inner region of the egg-shell catalyst particles existing in the remaining from the outer region of the geometric equivalent diameter.

3. The process according to any preceding claim, wherein the basic element is selected from alkali metals, alkaline earth metals, rare earth metals, and combinations thereof, preferably magnesium.

4. The process according to any preceding claim, wherein the egg-shell catalyst comprises, based on the total molar amount of gold, silicon, aluminum, the second element and the basic element: 0.03 mol% to 3 mol% of gold, 40 mol% to 90 mol% of silicon, 3 mol% to 40 mol% of aluminum, 0.1 mol% to 20 mol% of the second element and 2 mol% to 40 mol% of the basic element, and all these elements except for the gold and oxygen are present in the form of oxides.

5. The process according to any preceding claim, wherein the compound and the egg-shell catalyst share at least one same second element.

6. The process according to any preceding claim, wherein the compound is not the egg-shell catalyst.

7. The process according to any preceding claim, wherein the compound is added in portions or continuously, to the reaction mixture during the process.

8. The process according to any preceding claim, wherein the solubility of the compound in the reaction mixture under process conditions is at least 1 ppm, preferably at least 10 ppm, more preferably at least 100 ppm.

9. The process according to any preceding claim, wherein the compound comprises at least one salt of an inorganic or organic acid, and/or oxide, and/or hydroxide of the second element.

10. The process according to any preceding claim, wherein the mass ratio of the compound to the egg-shell catalyst present in the reactor mixture during the process is in the range 0.00001 to 1, preferably 0.0001 to 0.1.

11. The process according to any preceding claim, wherein the process is carried out continuously and the compound is continuously added to the process, wherein the weight ratio of the mass of the compound added to the reactor per time unit to the sum of the masses of all reactants per time unit is in the range between 0.1 ppm and 0.1 wt%.

12. The process according to claims 2-11, wherein the spent egg-shell catalyst, wherein the maximum Ni, Co, Fe, Zn and/or Ti concentration in an outer region of the spent egg-shell catalyst particles extending from the surface of the particles to 40% of an equivalent diameter thereof is less than 1,2 times as high as the maximum concentration of Ni, Co, Fe, Zn and/or Ti in the inner region of the spent egg-shell catalyst particles existing in the remaining from the outer region of the geometric equivalent diameter, is at least partially separated from the egg-shell catalyst, as defined in claim 2, and withdrawn from the reaction mixture and optionally replaced with the fresh egg-shell catalyst.

13. The process according to claim 13, wherein the spent egg-shell catalyst is separated using filtration and/or sedimentation.

14. The process according to claim 13 or 14, wherein the spent egg-shell catalyst is regenerated and optionally returned to the oxidative esterification process, wherein the regeneration is carried out by depositing Ni, Co, Fe, Zn and/or Ti precursors and optionally gold precursors on the outer shell of the spent egg-shell catalyst, followed by thermal decomposition of such precursors so that a (fresh) egg-shell catalyst is formed.

15. The process according to any preceding claim, wherein Ni, Co, Fe, Zn and/or Ti particles and/or ions finely dispersed and/or dissolved in the reaction mixture exiting the reactor for carrying out oxidative esterification reaction are trapped immediately after the reactor or further in the product workup, using a suitable trapping means, such as an ion exchange resin or an appropriate adsorbent, e.g. active carbon.
